# EUROPEAN PATENT SPECIFICATION

(11) **EP 3 916 164 B1**
(45) Date of publication and mention of the grant of the patent: **30.07.2025**
(21) Application number: 20708528.3
(22) Date of filing: 21.01.2020
(51) Int. Cl.: E03C 1/04

(54) **TAP WITH EXTERIOR GALVANIC PROTECTION**
WASSERMISCHER MIT GALVANISCHEM SCHUTZ
ROBINETTE AVEC UNE PROTECTION GALVANIQUE

(30) Priority: 23.01.2019 ES 201930108 U
(43) Date of publication of application: 01.12.2021
(73) Proprietor: Industrias Ramon Soler, S.A., 08970 Sant Joan Despi (ES)
(72) Inventor: FERRER-DALMAU NIETO, Francisco, 46021 Valencia (ES)
(74) Representative: Ponti & Partners, S.L.P
(86) International application number: PCT/ES2020/070044
(87) International publication number: WO 2020/152384

(56) References cited:
- EP-A1- 2 853 644
- EP-A2- 2 592 175
- WO-A1-2008/004933
- DE-A1- 4 404 194

## Description

The present invention relates to a tap, preferably for sanitary uses for potable water, and to the means for the galvanic protection of the constitution of the external structure thereof against the external corrosion of the tap and of the coatings applied thereto.

### Background of the invention

Means for the galvanic protection of the internal elements of water conduits are well known and are therefore part of the state of the art; these elements enter into contact with the water and the galvanic protection prevents their oxidation and the corresponding issues associated therewith, regarding both the useful life and the malfunctioning of the internal items in contact with the water, and the possible migration into the potable water of compounds resulting from the oxidation, with the corresponding associated issues.

The galvanic protection of said internal items is well known; this is achieved by means of the connection of materials acting as a sacrificial anode, always associated with those internal parts made of lead or aluminium and which present said issues of compound migration into the potable water.

For the external protection of the tap, the system habitually used is a zinc plating of between 8 - 15 microns, followed by a chromed outer coating for external mechanical protection and aesthetic purposes. The cost of said external protective coatings is considerable, with regard to the production cost of the tap, significant thicknesses being necessary in order to ensure a good external protection of the tap, particularly in locations with a greater risk of external corrosion, such as facilities installed in areas of high humidity and salinity, such as those near the coast.

Documents WO2008004933A and EP2853644A disclose water taps with galvanic protection with a sacrificial anode.

### Description of the invention

The object of the present invention is to provide a tap with galvanic protection of its external elements and which resolves the aforementioned drawbacks; and presenting other advantages to be described below.

In accordance with this object, and with regard to a first aspect, the present invention is based on a tap preferably for water-related installations such as sanitary fittings, kitchens, showers, etc., whose external parts are visible to the user and are therefore exposed to the environmental conditions of the location where they are installed. Said tap has at least one external galvanic protective coating of a particular thickness for the protection of said external parts.

The inventive tap comprises a connection between at least the external parts of the tap body and a sacrificial anode of anodic material with regard to the material of said external tap body parts, where the connection is performed at the base in a manner not visible to the user of the tap, wherein this base is used for installing the tap to the wall, sanitary fitting, worktop or similar.

This configuration enables the achievement of an advantageous reduction in the galvanic protection by way of a coating, generally using zinc at a thickness of between 8 and 15 microns, being significantly reduced in 30%, thanks to the auxiliary galvanic protection provided by the sacrificial anode which is installed on the external tap body parts.

This enables an ideal utilisation of the new tap in facilities under conditions of high humidity and salinity, with no need for over-protection with a greater zinc coating; even possibly reducing the same.

In accordance with the invention, the anodic material of which the sacrificial anode is comprised is Zinc, Magnesium, Aluminium or another metal less noble than the material of the external tap body, which is brass, steel and/or stainless steel.

The constitution of the anode using said materials ensures its function as a sacrificial anode in favour of the metallic material of which the tap is constructed, preferably brass or steel, maintaining this corrosion-free.

In one embodiment of the invention, the external parts of the tap body are not in contact with the water distributed via the internal parts of the tap there being at least intermediate elements.

This last enables the independent protection of taps featuring conduits that are independent from the external body and which may be made from other materials for the protection thereof against the action of the water.

As an alternative option to the above, the external tap body parts may be, at least to a certain extent, in contact with the water distributed through the interior of said tap.

### Brief description of the drawings

For the better understanding of the description made herein, a set of drawings has been provided wherein, schematically and solely by way of a non-limitative example, a practical case of an embodiment is portrayed.
Figure 1 is a perspective view from below of a tap showing the housing for the installation of the sacrificial anode.
Figure 2 is a detail view of the location for the installation of the sacrificial anode, and of the sacrificial anode in a position prior to its solidary installation in the base of the internal body of the tap.

### Description of a preferred embodiment

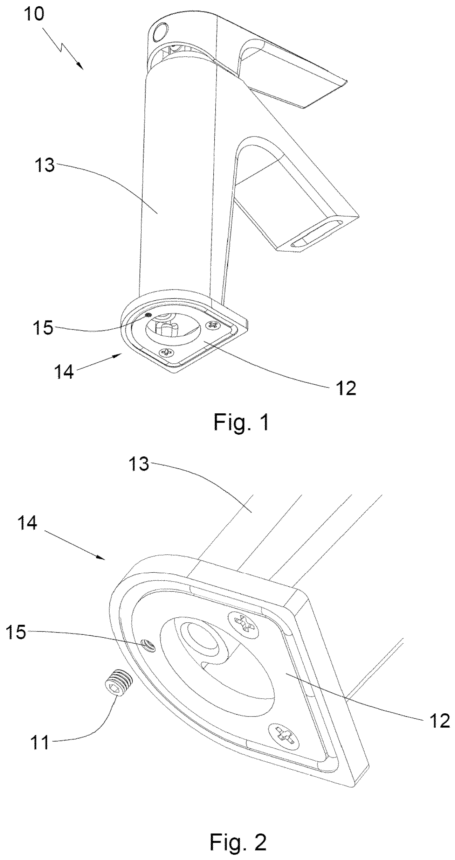

In the present preferred embodiment of the invention, and as is shown in figures 1 and 2, it is understood that the tap (10) with external galvanic protection by means of a sacrificial anode (11) is of the regular type commonly installed in potable water facilities such as toilets, kitchens, etc.

The tap (10) features a configuration based on a metallic body (12) of external parts, made of stainless steel in the present embodiment; said external parts, visible to the user, being covered by an external coating (13) forming an external galvanic protective layer on said visible external parts; in the present embodiment said layer is zinc with a thickness of approximately 5.5 microns.

In the present embodiment, the base (14) is used to couple the body (12), to the sanitary fitting or support thereof (10). The base (14) has connecting means (15), in this case formed by a threaded housing, wherein a sacrificial anode (11) of anodic material, in this case Magnesium, is fixed. The connecting means located at the base (14) are not visible to the user in normal use. As has been stated in the description of the invention, materials different from these may alternatively be used, both in the constitution of the body (12) of the tap (10) and in the external galvanic protection (13) and in the sacrificial anode (11)), provided that the differences in nobility of the protective materials employed are maintained, regarding that of the constitution of the body (12) of the tap (10).

The connection of the sacrificial anode (11) with the body (12) of the tap (10) with external parts is performed by means of direct contact between the two metals, and enables the external coating (13) to be approximately 30% less thick than in the case of the absence of a sacrificial anode (11), maintaining the same conditions of external galvanic protection. In the present embodiment, the connection is performed by means of a threaded coupling between the sacrificial anode (11) and the connection means (15), fixing its position and preventing the detachment thereof. Other alternative coupling means are possible, provided that they enable said contact between the metallic materials.

In the present embodiment, it is understood that the external parts of the body (12) of the tap (10) are not parts which enter into contact with the water for consumption; therefore, the sacrificial anode (11) does not act as galvanic protection for said parts which enter into contact with the water. Alternatively, these external parts of the body (12) of the tap (10) do coincide, at least to a certain extent, with parts which enter into contact with the water for consumption; the sacrificial anode (11) therefore does act as galvanic protection thereof, obviating new specific protection for said parts or improving their protection.

## Claims

1. A tap with external galvanic protection, the tap featuring a body (12) with external parts visible to the user, said external parts being covered by at least one external protective galvanic coating (13) protecting said external parts, the tap further comprising a base (14) for installing the tap to a wall, a sanitary fitting or to a worktop,
wherein
the tap (10) comprises a connection (15) between at least the external parts of the body (12) and a sacrificial anode (11), wherein the sacrificial anode (11) is made of anodic material with respect to the material of said external parts of the body (12);
wherein the connection (15) is executed at the base (14) in a manner not visible to the user of the tap in a normal use, the sacrificial anode (11) making contact with the external parts of the body (12); and
wherein the anodic material of which the sacrificial anode (11) is comprised is Zinc, Magnesium, Aluminium or another metal less noble than the material of the body (12) with external parts, which is brass, steel and/or stainless steel.

2. A tap with external galvanic protection, according to claim 1, wherein the parts of the body (12) of the tap (10) which are external do not enter into contact with the water distributed via the internal parts of the tap (10), there being at least intermediate elements.

3. A tap with external galvanic protection, according to claim 1, wherein the parts of the body (12) of the tap (10) which are external enter into contact, at least to a certain extent, with the water distributed via the internal parts of the tap (10).

## Patentansprüche

1. Wasserhahn mit externem galvanischen Schutz, wobei der Wasserhahn einen Körper (12) mit für den Benutzer sichtbaren äußeren Teilen aufweist, wobei die äußeren Teile durch mindestens eine externe schützende galvanische Schicht (13), die diese äußeren Teile schützt, bedeckt werden, wobei der Wasserhahn weiter eine Basis (14) für Montage des Wasserhahns an einer Wand, einem Sanitäranschlussstück oder einer Arbeitsplatte umfasst, wobei
der Wasserhahn (10) eine Verbindung (15) zwischen mindestens den äußeren Teilen des Körpers (12) und einer Opferanode (11) umfasst, wobei die Opferanode (11) aus anodischem Material in Bezug auf das Material der äußeren Teile des Körpers (12) hergestellt ist;
wobei die Verbindung (15) an der Basis (14) in einer für den Benutzer des Wasserhahns bei einem normalen Gebrauch nicht sichtbaren Weise ausgeführt ist, wobei die Opferanode (11) mit den äußeren Teilen des Körpers (12) in Kontakt kommt; und wobei das anodische Material, aus dem die Opferanode (11) aufgebaut ist, Zink, Magnesium, Aluminium oder ein anderes Metall ist, das weniger edel als das Material des Körpers (12) mit äußeren Teilen, die Messing, Stahl und/oder rostfreier Stahl sind, ist.

2. Wasserhahn mit externem galvanischen Schutz nach Anspruch 1, wobei die Teile des Körpers (12) des Wasserhahns (10), die außen sind, nicht mit dem Wasser in Kontakt kommen, das via die inneren Teile des Wasserhahns (10) verteilt wird, wobei es zumindest Zwischenelemente gibt.

3. Wasserhahn mit externem galvanischen Schutz nach Anspruch 1, wobei die Teile des Körpers (12) des Wasserhahns (10), die außen sind, zumindest bis zu einem gewissen Grad, mit dem via die inneren Teile des Wasserhahns (10) verteilten Wasser in Kontakt kommen.

## Revendications

1. Robinet avec protection externe galvanique, le robinet présentant un corps (12) avec des parties externes visibles pour l'utilisateur, lesdites parties externes étant recouvertes d'au moins un revêtement galvanique de protection externe (13) protégeant lesdites parties externes, le robinet comprenant en outre une base (14) pour installer le robinet sur un mur, un raccord sanitaire ou un plan de travail, dans lequel
le robinet (10) comprend un raccordement (15) entre au moins les parties externes du corps (12) et une anode sacrificielle (11), dans lequel l'anode sacrificielle (11) est réalisée en un matériau anodique par rapport au matériau desdites parties externes du corps (12) ;
dans lequel le raccordement (15) est exécuté au niveau de la base (14) d'une manière non visible pour l'utilisateur du robinet dans une utilisation normale, l'anode sacrificielle (11) venant en contact avec les parties externes du corps (12) ; et
dans lequel le matériau anodique dont l'anode sacrificielle (11) est composée du zinc, du magnésium, de l'aluminium ou un autre métal moins noble que le matériau du corps (12) avec des parties externes, qui est du laiton, de l'acier et/ou de l'acier inoxydable.

2. Robinet avec protection galvanique externe, selon la revendication 1, dans lequel les parties du corps (12) du robinet (10) qui sont externes ne viennent pas en contact avec l'eau distribuée via les parties internes du robinet (10), au moins des éléments intermédiaires étant présents.

3. Robinet avec protection galvanique externe, selon la revendication 1, dans lequel les parties du corps (12) du robinet (10) qui sont externes viennent en contact, au moins dans une certaine mesure, avec l'eau distribuée via les parties internes du robinet (10).
